# EUROPEAN PATENT APPLICATION

(11) **EP 3 786 618 A1**
(43) Date of publication of application: **03.03.2021**
(21) Application number: 19193619.4
(22) Date of filing: 26.08.2019
(51) Int. Cl.: G01N 21/3577, G01N 33/50

(54) **A METHOD FOR ANALYZING A PERITONEAL DIALYSIS SAMPLE**

(71) Applicant: Veterinärmedizinische Universität Wien, 1210 Vienna (AT); Medizinische Universität Wien, 1090 Wien (AT)
(72) Inventor: Grunert, Tom, 1150 Wien (AT); Kratochwill, Klaus, 1200 Wien (AT)
(74) Representative: Nemec, Harald

(57) **Abstract**

A method for analyzing a peritoneal dialysis analysis sample comprising the steps of
a) providing an analysis sample from a subject, wherein the subject is subjected to peritoneal dialysis and the analysis sample is based on the peritoneal dialysis effluent of said subject,
b) measuring a sample spectrum of analysis sample in a spectral range of from 4000 cm⁻¹ to 400 cm⁻¹ in a spectroscopy step applying Fourier-Transform-Infrared spectroscopy,
c) determining, in a comparison step, a similarity value by comparing said sample spectrum to at least one reference spectrum obtained from at least one reference sample by measuring as defined in step b),
d) assigning the analysis sample to a clinical parameter based on said similarity value.

## Description

The present invention relates to a method for analyzing a peritoneal dialysis sample.

### Background of the invention

Patients with end-stage renal disease usually depend on life-saving dialysis for removal of salts and water from the uremic body. Peritoneal dialysis (PD) offers specific advantages, such as more freedom from hospital-based dialysis centers, better preservation of residual renal function and an advantage in survival at the beginning of renal replacement therapy.

Unfortunately, these benefits are counterbalanced by limitations of PD, including risk of peritonitis, inflammation and membrane failure as main physiological culprits of technique failure. Occurrence of bacterial infections requires rapid and accurate decisions based on profound diagnostics.

The events that trigger an undesired termination of the PD may be classified in three categories: i) the patient has a peritonitis (i.e. an inflammatory event), ii) the patient has a peritoneal membrane deterioration or iii) a technical failure occurs, wherein technical failure is a collective term for events leading to undesired termination of PD therapy (including transplantation, death, switch to hemodialysis, etc.).

Management of PD patients currently mainly relies on systemic parameters (i.e. routine laboratory assessment of blood, plasma or serum) and the peritoneal equilibration test (PET) which is a functional test of peritoneal transport and assesses local/peritoneal parameters. Systemic parameters might be diagnostically conclusive too late and too unspecific. The PET is a laborious, time consuming, and cost intensive way of guiding the therapy regime of PD patients. Long-term surveillance of patients on home therapy, having only intermittent routine checks at the tertiary care center, is also a challenge in PD patients, often leading to late discovery of complications.

In peritoneal dialysis, a specific solution (PD fluid) is introduced in the lower abdomen (i.e. the abdominal cavity) and the peritoneum acts as membrane for exchange with the blood. Subsequently, the introduced volume is removed from the abdominal cavity. The removed solution is the so-called PD effluent. Thus, the PD effluent is a fluid directly obtained from the subject's body. The PD effluent contains significant information on peritoneal biology and state of the membrane in form of dissolved molecules.

Various studies enabled deeper characterization of PD effluent on the level of contained proteins, metabolites and cells (Kratochwill 2016, Aufricht 2017, Herzog 2017, Herzog 2018, Wiesenhofer 2019), none of which is accepted yet as predictive biomarker (Aufricht 2017). While these methods allow broader biological insight, they are substantially more expensive and time-consuming than clinically applicable diagnostic tests.

Spectroscopic methods have been suggested for diagnosing and analyzing in the context of dialysis. US 2004/0204634 suggests a method for monitoring hemodialysis including obtaining a Raman spectrum of a sample, i.e. patient's blood. Raman spectroscopy is a scattering technique. A molecule showing Raman activity requires changes in polarizability. Another Raman-based method is described in WO 2015/164620 A1 (US 2017/045455 A1). Therein, the method requires to determine an analyte pattern of at least two analytes present in a sample. In the example, the sample is a dialysate, which occurs in hemodialysis.

So far, no method for analyzing a PD patient's biofluid has been disclosed, which allows to assign a clinical parameter in order provide clinicians with better tools to monitor / manage dialysis regime and/or to predict the PD outcome.

### Short description of the invention

The present invention provides a method for analyzing a peritoneal dialysis analysis sample comprising the steps of
a) providing an analysis sample from a subject, wherein the subject is subjected to peritoneal dialysis and the analysis sample is based on the peritoneal dialysis effluent of said subject,
b) measuring a sample spectrum of said sample in a spectral range of from 4000 cm⁻¹ to 400 cm⁻¹ in a spectroscopy step applying Fourier-Transform-Infrared (FTIR) spectroscopy,
c) determining, in a comparison step, particularly in a computing step, a similarity value by comparing said sample spectrum to at least one reference spectrum obtained from at least one reference sample by measuring as defined in step b),
d) assigning the analysis sample to a clinical parameter based on said similarity value.

The inventors found that FTIR-technology allows to analyze a PD effluent sample and to predict, i.e. assign, parameters with clinical importance in a straight forward way. A pilot study shows that even based on a small dataset, the method allowed to assign PD-related parameters and physiological parameters such as concentration of immune-related biomarkers with high accuracy (Example 1).

Thus, the method according to the invention provides a rapid, non-invasive and cost effective tool to obtain clinical parameters of importance within PD therapy, which allows to assess the patient status and guide PD management.

Compared to conventionally used molecular tests, e.g. enzyme-linked immunosorbent assay (ELISA), a chemometric-assisted spectroscopy method has many advantages since it relies on a "holistic view" of a sample via the simultaneous investigation of a range of different molecules instead of identifying and quantifying specific compounds. With FTIR-technology a spectral picture of the overall chemical composition of the main biochemical constituents in the sample is generated (also referred as molecular spectral fingerprint).

Since FTIR also allows high-throughput analytics at low running costs (reagent-free, label-free, reusable sample holders), it is perfectly suited to large-scale studies as well as to translation to clinically usable routine analytics undertaken daily.

### Detailed description of the invention

In the method according to the invention, a "sample" is based on a peritoneal dialysis effluent (PDE) from a subject receiving PD. As the PDE is a byproduct in PD therapy, the sample collection is straight-forward and convenient for the patient.

In a preferred embodiment, the sample is the peritoneal dialysis effluent.

Alternatively, the PDE may be subjected to a sample preparation, wherein the preparation may include steps as e.g. centrifugation and separating, diluting, freezing, thawing cooling, and/or drying. The sample may be prepared as liquid or as dry sample.

Accordingly, in another embodiment, the sample is prepared from the peritoneal dialysis effluent, in particular prepared by diluting the peritoneal dialysis effluent and/or freezing and thawing the peritoneal dialysis effluent.

As used herein, the term "analysis sample" refers to the sample to be analyzed by the method of the present invention and provided in step a).

On the other hand, as used herein, the "at least one reference sample" refers to a sample for comparison.

The analysis sample and the reference sample, both, are based on the PDE of a subject. Accordingly, the embodiments and preference as to preparing the analysis sample apply for the reference sample either independently or analogously. Preferably, the at least one reference sample is prepared in analogy to the analysis sample, i.e. applying the same or similar preparation steps.

In certain embodiments, the reference sample is based on the PDE
- of a PD subject different to the subject from which the PDE for the analysis sample is taken (i.e. reference sample from a reference subject) or
- of the same subject from which the PDE for the analysis sample is taken.

In the first case, the method relies on differences (or similarities) between individual subjects with different physiological and/or pathological states.

In the second case, it is particularly preferred that the reference sample is from the same subject but taken at a different time point. Thus, the method relies on different states a subject may have over time. The second case is of special interest to imply a longitudinal comparison. In case of longitudinal comparison, also the relative differences to the reference sample over time may allow to determine the similarity value and assign the clinical parameter, in particular with an algorithm trained with pairs of reference samples taken at different time points.

Preferably, the reference sample is related (or pre-assigned) to a certain clinical parameter. The assignment or relation of a reference sample to a defined clinical parameter may be done by conventional diagnostics, e.g. including evaluation of systemic parameters, PET data, medical history, and medical documentation.

In step b) of the method according to the invention, the analysis sample is subjected to measuring a spectrum in a spectral range of from 4000 cm⁻¹ to 400 cm⁻¹ in a spectroscopy step applying Fourier-Transform-Infrared (FTIR) spectroscopy.

The sample spectrum and the reference spectrum are measured independently to each other based on the analysis sample and the reference sample, respectively. However, the reference sample is measured as defined in step b), accordingly the reference spectrum is measured in analogy to the sample spectrum. Thus, the at least one reference spectrum is measured applying the same or similar measuring conditions (i.e. technique, spectral range, ...) and the embodiments relating to the sample spectrum analogously apply for the at least one reference spectrum.

The term "FTIR spectroscopy" refers to an absorption spectroscopic technique. FTIR depends on changes in the dipole moment of a molecule (in contrast to Raman spectroscopy). According to the FTIR principle, simultaneously high-spectral-resolution data over a spectral range in the infra-red region are collected and processed by Fourier transformation.

As used herein, FTIR spectroscopy includes FTIR transmission sample technique as well as FTIR attenuated total reflection (FTIR-ATR). Both techniques rely on the FTIR principle. Difference between transmission sample technique and ATR is in the optical path the light takes during measurement in respect to the sample. In FTIR transmission sample technique, the light transmits the sample. Thus, thin samples such as dried samples from a liquid or fluid are preferred or required. With ATR technique, the light is reflected at the sample and transmission is not required. Thus, both liquid or dried samples may be analyzed with ATR. In the method according to the present invention, preparation as liquid sample may be preferred as the PDE itself is liquid and drying as time-consuming preparation step can be omitted, if the sample is liquid. On the other hand, in contrast to the liquid sample, a dried sample has a higher concentration of the included biomolecules and a better signal intensity may be obtained.

Accordingly, in one embodiment, said spectrum in step b) is obtained by applying FTIR transmission sample technique, wherein in step a) the sample is dried. In another embodiment, said spectrum in step b) is obtained by FTIR attenuated total reflection (ATR), wherein in step a) the sample is provided as liquid or dried.

The term "spectrum" as used herein refers to an optical spectrum. As understood herein, the term spectrum comprises one or more sample spectral components, the spectral components comprising a wavenumber and an absorbance value. In the spectroscopy step b) a plurality of optical values is determined in the spectral range, wherein each of the optical values of the plurality is related to a certain wave number. Thus, any spectrum may be a data set including multiple pairs of data points (wavenumber/absorbance value), i.e. a function.

According to the present invention, the sample spectrum is measured within the spectral region of from 4000 cm⁻¹ to 400 cm⁻¹. Within this range, certain subranges are of special interest because they relate to biomolecules. The range of from 3000 cm⁻¹ to 2800 cm⁻¹ is of interest because of signals related to fatty acids occur in this spectral range (fatty acid region). The range of 1500 cm⁻¹ to 1200 cm⁻¹ is of interest because of signals related to proteins and fatty acids occur in this spectral range (mixed region). The range of 1800 cm⁻¹ to 1500 cm⁻¹ is of interest because of signals related to proteins occur in this spectral range (protein region). The range of from 1200 cm⁻¹ to 800 cm⁻¹ is of interest because of signals related to carbohydrates occur in this spectral range (polysaccharide region). Preliminary data identified the range of from 1800 cm⁻¹ to 1500 cm⁻¹ or from 1200 cm⁻¹ to 800 cm⁻¹ as preferred regions for analysis of PDE based samples (Example 1).

Accordingly, in one embodiment, in step b), the sample spectrum is measured in at least one of the spectral ranges selected from the group consisting of from 3000 cm⁻¹ to 2800 cm⁻¹, from 1500 cm⁻¹ to 1200 cm⁻¹, from 1800 cm⁻¹ to 1500 cm⁻¹ and from 1200 cm⁻¹ to 800 cm⁻¹, preferably in the range of from 1800 cm⁻¹ to 1500 cm⁻¹ or from 1200 cm⁻¹ to 800 cm⁻¹.

In step c), a similarity value is determined by comparing said sample spectrum to at least one reference spectrum.

In a certain embodiment, in step c) the comparison step includes pre-processing of the sample spectrum, the reference spectrum, or both.

As understood here, "pre-processing" may include calculating the n^{th} derivative of a spectrum, where n is a natural number. In the context of the present specification, the term derivative is used in its meaning known in the art of mathematics. The term "derivative of a spectrum" denotes a derivative of a function, wherein the independent variable is a wavelength or a wave number, and wherein the dependent variable is an optical value, and wherein the optical values determined by a spectroscopy measurement of a sample are approximated by the function. In certain embodiments, the first, second, third, fourth or fifth derivative, preferably the second derivative is calculated.

Pre-processing may further include normalization of a spectrum, preferably vector normalization. Therein, the vector normalization is performed as follows: A plurality of optical values is determined from a liquid sample, wherein each optical value is determined at a specific wave number. Subsequently, a function is provided, wherein the function comprises a plurality of x-values, wherein each x-value corresponds to a wave number, and a plurality of y-values, wherein each y-value corresponds to an optical value, and wherein the function describes the relationship between the x-values and the y-values. Furthermore, a mean value of the plurality of y-values is determined, and the mean value is subtracted from each y-value, resulting in a plurality of corrected y-values. Subsequently, the square of each corrected y-value is calculated, and the sum of all squares is determined. Finally, each corrected y-value is divided by the sum of all squares, resulting in a plurality of vector normalized y-values, wherein the vector normalized y-values and the wave numbers, at which the respective y-value were determined, constitute a vector normalized spectrum. Advantageously, vector normalization allows better analysis of spectra, particularly comparison of spectra.

In one embodiment, the pre-processing is selected from the group consisting of
- Savitzky-Golay smoothing,
- multiplicative scatter correction (MSC),
- standard normal variate (SNV),
- spectral differentiation, such as first-, second-, third-, fourth- or fifth order differentiation
- baseline correction, such as rubber band, automatic weighted least squares, Whittaker filter
- normalization,
- scaling, such has mean-centering, block scaling, and
- combination thereof.

In a preferred embodiment, the pre-processing includes calculating the second derivative of the spectrum and subsequent vector normalization of the derivative to obtain a derived vector normalized spectrum.

For determining the similarity value, the analysis spectrum is compared with at least one reference spectrum measured as defined above. In certain embodiments, the at least one reference spectrum is stored as sets of data. In this manner, the reference spectrum may be used several times to be compared to sample spectra of different analysis samples.

It is preferred that the reference spectrum is pre-processed in analogy to the sample spectrum. In this embodiment, it may be preferred that the pre-processing is not performed in step c) but the at least one reference spectrum is stored being pre-processed, e.g. as derived vector normalized reference spectrum.

The "similarity value" as used herein is the result from a comparison step, in particular a computing step, wherein the analysis sample is directly or indirectly compared to at least one reference sample. For example, the similarity value is larger if the sample spectrum is more similar to the reference spectrum. Depending on the comparison step, the similarity value may be a real number or a natural number. The similarity value may also be a category, e.g. "yes/no", or a numerical value translated to a category, e.g. "low", "high" or "no result".

Accordingly, in certain embodiments, the similarity value is a numerical variable and/or categorical variable.

In certain embodiments, the similarity value is determined by calculating a statistical parameter of the analysis spectrum, particularly a univariate statistical parameter, more particularly a mean or a median, or a multivariate statistical parameter, and comparing a reference spectrum to the statistical parameter.

In certain embodiments, an algorithm is used to determine the similarity value in the comparison step, particularly in a computing step, wherein the algorithm includes application of a machine learning model.

Algorithms based on machine learning models are preferred algorithms as they may be of dynamic nature. I.e. the set of reference samples may be expanded incrementally to further improve the algorithm over time.

The person skilled in the art may select the method for computing the similarity value depending on the nature of the clinical parameter, the amount and quality of reference sample. For example, in certain embodiments, the machine learning model is selected from the group consisting of
- neural networks, such as Artificial Neural Network (ANN), Convolutional Neural Network (CNN)
- support vector machines,
- discriminant analysis, such as linear/ quadratic/ Mahalanobis discriminant analysis; partial least square discriminant analysis; canonical variates analysis; discriminant function analysis,
- k-nearest neighbors algorithm (also abbreviated as k-NN),
- regression analysis, such as linear regression, multiple linear regression, principal component regression, partial least square regression analysis, logistic regression,
- evolutionary-based algorithms, such as genetic algorithm, genetic programming/computing, evolutionary programming,
- regression and decision tree learning, such as classification and regression tree (CART), boosted trees; bootstrap aggregated decision trees including random forests,
- adaptive boosting, and
- combination thereof.

In a more specific embodiment, the algorithm is based on principal component analysis-linear discriminate analysis (PCA-LDA) or principal component analysis-Mahalanobis discriminate analysis (PCA-MDA).

As shown in the example, clinical parameters could successfully be assigned to the investigated samples based on determining the similarity value calculating the principle components (PCA), i.e. a multivariate parameter of the pre-processed spectrum (second derivatives, normalized) and applying an algorithm based on linear discriminate analysis (LDA) or Mahalanobis discriminate analysis (MDA).

A machine learning model is created via a process called training (supervised or unsupervised). In a method of the present invention, the training may rely on a set of reference spectra. The set of reference spectra is obtained by measuring a plurality of reference samples as defined in step b). I.e. the set of reference spectra includes more than one reference spectrum. The reference spectra are measured from reference samples analogously or similarly to the sample spectrum. Preferably, each reference sample is a sample, wherein a certain clinical parameter is defined.

In embodiments using an algorithm, e.g. based on a machine learning model, in the computing step c), the algorithm (or the machine learning model) is trained using a set of reference spectra.

When a machine learning model is applied in step c), the similarity value is determined as output value of the model. In these cases, the comparison of the sample spectrum to at least one reference spectrum may be indirect. The machine learning algorithm includes the information from the reference spectrum during training and subsequently applies it, when computing the similarity value based on the (processed) sample spectrum.

In step d), the analysis sample is assigned to a clinical parameter based on said similarity value determined in step c). Thus, the result of the method for analyzing a peritoneal dialysis analysis sample is the prediction of a clinical parameter for said analysis sample.

The term "clinical parameter" as used herein, includes demographic parameters, physiological and pathological parameters including biomarker concentration(s), dialysis-related parameters, and outcome parameters.

It is to be understood that the clinical parameter assigned in step d) is of the same nature as the certain clinical parameter pre-assigned to the reference sample(s). Thus, the clinical parameter that may be analyzed for the analysis sample depend on the pre-assignment of the at least one reference sample or the set of reference spectra used for training the algorithm.

In a preferred embodiment, in step d), a clinical parameter is assigned selected from the group of physiological and pathological parameters including biomarker concentration(s), dialysis-related parameters, and outcome parameters. Assignment of such clinical parameter by a method based on FTIR technology allows to provide clinicians with faster access to parameters in comparison to other methods. Moreover, complex outcome parameters will allow to guide clinicians (and/or patients) in decision making during PD.

Furthermore, other descriptive demographic parameters, such as age, were shown to have a discriminative effect on the PDE spectrum (Example 1). Accordingly, such parameters could additionally be included in the step of determining the similarity value and/or they may be assigned with the method according to the present invention for control.

Accordingly, in one embodiment, the clinical parameter is selected from the group consisting of dialysis regime (e.g. automated cycler PD versus manual CAPD), duration of PD, co-morbidity, blood pressure, body-mass index, and age.

In one embodiment, the clinical parameter is a dialysis-related parameter selected from the group of residual urine output, ultrafiltration, residual clearance, dialysate-to-plasma creatinine ratio, residual glomerular filtration rate, peritoneal small solute transport rate, mass transfer area coefficient, effective lymphatic absorption rate, transcapillary ultrafiltration rate, free water transport, and sodium dip.

In another embodiment, the clinical parameter is a biomarker concentration, wherein the biomarker is selected from the group of proteins, such as cytokines and chemokines (e.g. interleukin-8, interleukin-6) and heat shock proteins (HSPs; e.g. Hsp72), or metabolites, such as amino acids (e.g. glutamine), and biogenic amines (e.g. methionine-sulfoxide).

In another embodiment, the clinical parameter is an outcome parameter indicating
- the subject's risk of having or developing a peritonitis,
- the subject's risk of having or developing a peritoneal membrane deterioration and/or
- the risk of technical failure.

Accordingly, the method according to the invention allows to analyze a peritoneal dialysis sample for prediction of the risk category in regard of the outcome of the peritoneal dialysis. The outcome may be the subject's risk of having or developing a peritonitis, the subject's risk of having or developing a peritoneal membrane deterioration and /or the risk for the technical failure of the peritoneal dialysis.

The invention is illustrated further by the following examples, which are not to be construed as limiting the invention to the specific procedures described therein.

### Examples

### Example 1 - Assignment of dialysis-related parameters and biomarker concentrations

### Patients

PD effluent samples were obtained during a prospective randomized, open-label, two-period, cross-over phase I/II study conducted at the Medical University of Vienna (Austria) at the Department of Nephrology. The study and study protocol was approved by the local ethics committee of the Medical University of Vienna (EK 867/2010, EK 1167/2013, EK 2035/2015) and registered at www.clinicaltrials.gov (NCT01353638). The study was performed in accord with the Declaration of Helsinki. All patients provided written informed consent prior to trial participation. The study design, eligibility criteria, randomization, clinical methods, patient characteristics and adverse events have been previously described (Kratochwill 2016). In brief, 20 stable PD patients (13 male/7 female mean age 58 years) with a mean PD vintage of 2.4 years were treated per protocol. Patients were judged as clinically stable and had no severe concomitant disease (5 patients had a history of peritonitis more than 3 months prior to sample collection).

Effluent samples were collected in standard collection tubes without any anticoagulants (Vacuette, Bio-Greiner-One, Kremsmünster, Austria) immediately after completion of instillation of the dialysis fluid into the patients' cavity (= time point 0) and 4 hours later (= time point 4 h) after each of two standard peritoneal equilibration tests, one using commercially available PD fluid (Dianeal, 3.86% glucose, Baxter, Deerfield, IL, USA) and a second with the same PD fluid supplemented with 8 mM alanyl-glutamine (Ala-Gln) (N(2)-alanyl-L-glutamine 200 mg/mL, Fresenius Kabi, Bad Homburg, Germany), performed in randomized order. The two PETs were separated by a wash-out period of 28 to 35 days.

### Sample collection and preparation

PD effluent was centrifuged (250 × g, 10 min) immediately following collection, and cell-free supernatant samples were aliquoted and stored at -80°C until further analysis. Biochemical measurements and transfer kinetics between peritoneal and systemic circulation were analyzed in serum (at 2 hours of PET) and dialysate specimens by standard methods in the clinical laboratory of the Vienna General Hospital. PD effluent concentrations of creatinine were determined by a kinetic measurement of Jaffé reaction and corrected for high glucose levels by determination of a correction factor from measurements of unused PD fluid with the same method. PD effluent interleukin 6 (IL-6) and interleukin 8 (IL-8) concentrations were measured with the Immulite system (Siemens,Vienna, Austria) or by Bio-Plex bead array (Bio-Rad,Hercules, CA).

### Sample preparation and FTIR spectroscopy

PD effluent samples were put in randomized order and were measured and analyzed blinded. First, PD effluent samples were thawed and 30 µL of undiluted or 1:2 dilutions in 0.9% NaCl were subsequently spotted on a zinc selenide (ZnSe) optical plate and dried at 40°C for 30 min. Infrared spectral acquisition was performed in transmission mode using a HTS-XT microplate adapter coupled to a Tensor 27 FTIR spectrometer (Bruker Optics GmbH, Ettlingen, Germany) using the following parameters: 4000 to 500 cm⁻¹ spectral range, 6 cm⁻¹ spectral resolution, zero-filling factor 4, Blackmann-Harris 3-term apodization and 32 interferograms were averaged with background subtraction for each spectrum (Grunert 2013). In total, 79 spectra (0 h effluent, n=39; 4 h effluents, n=40) were recorded derived from PD effluent samples of 20 patients.

### Spectral preprocessing and analysis

Spectral preprocessing and subtractive spectral analysis were performed using the software OPUS 7.2 (Bruker Optics GmbH). To increase the robustness of subsequent chemometric analysis, spectral preprocessing was performed to increase spectral resolution, to minimize baseline shifts and to adjust biomass variations among different sample preparations. Raw absorbance spectra were preprocessed for the whole spectral range (4000 - 500 cm⁻¹) either by (1) vector normalization followed by baseline correction or (2) by second derivatives of the original spectra with a 9-smoothing point Savitzky-Golay filter followed by vector normalization, the later method was used for the calculation of subtractive spectra, unsupervised chemometric analysis and supervised classifications (Grunert 2014, Grunert 2016).

Classification of spectroscopic data were performed using the highly discriminatory protein (1800-1500 cm⁻¹) or polysaccharide (1200-800 cm⁻¹) spectral region applying the following supervised learning based classification algorithms: principal component analysis-linear discriminate analysis (PCA-LDA), principal component analysis-Mahalanobis discriminate analysis (PCA-MDA).

### Unsupervised and supervised chemometrics

All multivariate analyses were performed using the software Unscrambler X (CAMO Software, Oslo, Norway). Unsupervised chemometrics was performed on preprocessed data employing principal component analysis (PCA).

PCA computation was based on the singular value decomposition (SVD) algorithm and either performed at the spectral range of proteins (1800-1500 cm⁻¹) or polysaccharides (1200-800 cm⁻¹). The score plot was used for explorative data analysis and shows possible discriminations between classes, whereas the loading plot provides information about relevant spectral regions.

Supervised chemometrics was performed on preprocessed data employing principal component analysis-linear discriminate analysis (PCA-LDA) and principal component analysis-Mahalanobis discriminate analysis (PCA-MDA) analysis. The linear method of discriminant analysis (LDA) is applied when the difference between the two classes is expressed by a linear function, whereas the Mahalanobis method (MDA) uses ellipses to define the distances. For PCA-LDA and PCA-MDA the following parameters were used: 7 components for PCA projection, assume equal prior probabilities and all variables have equal weights.

Samples were split in two different data sets were used: either (1) PD effluent samples without Ala-Gln after 4 hours dwell time (n=20) to reflect conditions during daily PD routine, or (2) samples from both treatments without and with Ala-Gln after 4 hours dwell time were combined (n=40) to obtain sufficient amount of data per group to run PCA-LDA and PCA-MDA analysis.

### Clinical parameters

All available numeric datasets relating to clinical parameters were divided into two balanced groups and transformed in category variables to achieve a binary classification problem, which allows categorial similarity values for assignment. The classification rate (%) was used as a classification performance measure, which is defined as the average between the individual accuracy (%) of each of the two classes which accounts for imbalanced datasets instead of counting the overall correct guesses (Gajjar 2013).

### Results

All results for principal component analysis-linear discriminate analysis (PCA-LDA) and principal component analysis-Mahalanobis discriminate analysis (PCA-MDA) were plotted as confusion matrix and all total accuracies are summarized in the following tables, wherein the abbreviations indicate Treatment A: PD fluid with 8 mM Ala-Gln supplementation, treatment B: standard PD-fluid, CAPD: continuous ambulatory peritoneal dialysis, UF: ultrafiltration, IL: interleukin, D/P Crea: dialysate-to-plasma creatinine ratio, Glu: history of dialysis with glucose based PDF only, Ico: history of dialysis with icodextrin-based PDF.

**Table 1. Classification rates (%) for patient-related parameters**

| **Spectral range** | **Proteins 1800-1500 cm⁻¹** | | | | **Polysaccharides and phosphorous-containing molecules 1200-800 cm⁻¹** | | | |
|---|---|---|---|---|---|---|---|---|
| **Parameter** | **Model** | | | | | | | |
| **Age (< 60/ ≥ 60)** | **PCA-LDA** | | **PCA-MDA** | | **PCA-LDA** | | **PCA-MDA** | |
| **Treatment B, 4h (n=20)** | | | | | | | | |
| Predicted | Actual | | Actual | | Actual | | Actual | |
| | >60 | <60 | ≥60 | <60 | ≥60 | <60 | ≥60 | <60 |
| ≥60 | 8 | 2 | 10 | 0 | 10 | 1 | 10 | 0 |
| <60 | 2 | 8 | 0 | 10 | 0 | 9 | 0 | 10 |
| Accuracy % | 80.0 | 80.0 | 100.0 | 100.0 | 100.0 | 90.0 | 100.0 | 100.0 |
| **Overall accuracy %** | | **80.0** | | **100.0** | | **95.0** | | **100.0** |

| **Treatment A+B, 4h (n=40)** | | | | | | | | |
|---|---|---|---|---|---|---|---|---|
| Predicted | Actual | | Actual | | Actual | | Actual | |
| | ≥60 | <60 | ≥60 | <60 | ≥60 | <60 | ≥60 | <60 |
| ≥60 | 17 | 2 | 18 | 2 | 13 | 2 | 20 | 1 |
| <60 | 3 | 18 | 2 | 18 | 7 | 18 | 0 | 19 |
| Accuracy % | 85.0 | 90.0 | 90.0 | 90.0 | 65.0 | 90.0 | 100.0 | 95.0 |
| **Overall accuracy %** | | **87.5** | | **90.0** | | **77.5** | | **97.5** |

| **Parameter** | **Model** | | | | | | | |
|---|---|---|---|---|---|---|---|---|
| **Sex** | **PCA-LDA** | | **PCA-MDA** | | **PCA-LDA** | | **PCA-MDA** | |
| **Treatment B, 4h** | | | | | | | | |
| Predicted | Actual | | Actual | | Actual | | Actual | |
| | woman | man | woman | man | woman | man | woman | man |
| woman | 5 | 2 | 2 | 0 | 5 | 2 | 3 | 0 |
| man | 2 | 11 | 5 | 13 | 2 | 11 | 4 | 13 |
| Accuracy % | 71.43 | 84.62 | 28.57 | 100.00 | 71.43 | 84.62 | 42.86 | 100.00 |
| **Overall accuracy %** | | **78.0** | | **64.3** | | **78.0** | | **71.4** |

| **Treatment A+B, 4h** | | | | | | | | |
|---|---|---|---|---|---|---|---|---|
| Predicted | Actual | | Actual | | Actual | | Actual | |
| | woman | man | woman | man | woman | man | woman | man |
| woman | 9 | 5 | 9 | 2 | 7 | 8 | 8 | 4 |
| man | 5 | 21 | 5 | 24 | 7 | 18 | 6 | 22 |
| Accuracy % | 64.29 | 80.77 | 64.29 | 92.31 | 50.00 | 69.23 | 57.14 | 84.62 |
| **Overall accuracy %** | | **72.5** | | **78.3** | | **59.6** | | **70.9** |

| **Parameter Therapy (CAPD/ Cycler)** | **Model** | | | | | | | |
|---|---|---|---|---|---|---|---|---|
| | **PCA-LDA** | | **PCA-MDA** | | **PCA-LDA** | | **PCA-MDA** | |
| **Treatment B, 4h** | | | | | | | | |
| **Spectral range** | **Proteins 1800-1500 cm⁻¹** | | | | **Polysaccharides and phosphorous-containing molecules 1200-800 cm⁻¹** | | | |
| Predicted | Actual | | Actual | | Actual | | Actual | |
| | CAPD | Cycler | CAPD | Cycler | CAPD | Cycler | CAPD | Cycler |
| CAPD | 8 | 2 | 10 | 2 | 9 | 0 | 9 | 0 |
| Cycler | 2 | 8 | 0 | 8 | 1 | 10 | 1 | 10 |
| Accuracy % | 80.0 | 80.0 | 100.0 | 80.0 | 90.0 | 100.0 | 90.0 | 100.0 |
| **Overall accuracy %** | | **80.0** | | **90.0** | | **95.0** | | **95.0** |

| **Treatment A+B, 4h** | | | | | | | | |
|---|---|---|---|---|---|---|---|---|
| Predicted | Actual | | Actual | | Actual | | Actual | |
| | CAPD | Cycler | CAPD | Cycler | CAPD | Cycler | CAPD | Cycler |
| CAPD | 18 | 2 | 18 | 2 | 16 | 3 | 20 | 1 |
| Cycler | 2 | 18 | 2 | 18 | 4 | 17 | 0 | 19 |
| Accuracy % | 90.0 | 90.0 | 90.0 | 90.0 | 80.0 | 85.0 | 100.0 | 95.0 |
| **Overall accuracy %** | | **90.0** | | **90.0** | | **82.5** | | **97.5** |
| **Parameter** | **Model** | | | | | | | |
| **Glucose/ Icodextrin** | **PCA-LDA** | | **PCA-MDA** | | **PCA-LDA** | | **PCA-MDA** | |

| **Treatment A+B, 4h** | | | | | | | | |
|---|---|---|---|---|---|---|---|---|
| Predicted | Actual | | Actual | | Actual | | Actual | |
| | Glu | Ico | Glu | Ico | Glu | Ico | Glu | Ico |
| Glu | 26 | 0 | 32 | 1 | 28 | 1 | 32 | 2 |
| Ico | 6 | 8 | 0 | 7 | 4 | 7 | 0 | 6 |
| Accuracy % | 81.25 | 100.00 | 100.00 | 87.50 | 87.50 | 87.50 | 100.00 | 75.00 |
| **Overall accuracy %** | | **90.6** | | **93.8** | | **87.5** | | **87.5** |
| **Parameter** | **Model** | | | | | | | |
| **Time at PD** | **PCA-LDA** | | **PCA-MDA** | | **PCA-LDA** | | **PCA-MDA** | |

| **Treatment A+B, 4h** | | | | | | | | |
|---|---|---|---|---|---|---|---|---|
| Predicted | Actual | | Actual | | Actual | | Actual | |
| | <1y | ≥1y | <1y | ≥1y | <1y | ≥1y | <1y | ≥1y |
| <1y | 8 | 4 | 7 | 3 | 8 | 5 | 8 | 1 |
| ≥1y | 2 | 26 | 3 | 27 | 2 | 25 | 2 | 29 |
| Accuracy % | 80.00 | 86.67 | 70.00 | 90.00 | 80.00 | 83.33 | 80.00 | 96.67 |
| **Overall accuracy %** | | **83.3** | | **80.0** | | **81.7** | | **88.3** |

Classification results based on FTIR-spectroscopic data related to the patient characteristic, age and sex, are shown in table 1. High classification rates (≥ 90%) were obtained for both datasets (n=20 and n=40) for patient age (higher 60/ below 60) using PCA-MDA in either the protein or polysaccharide spectral region. Discrimination between PD samples derived from female and male patients revealed no favorite spectral range or classifier and had a maximum of 78.3 % total accuracy (n=40, PCA-MDA at the protein region). Thus, FTIR spectroscopic data modelling provide evidence for the existence of an age-related molecular signature of PD effluent samples capable to discriminate between patients younger or older than 60 years.

Further demographic data related to the therapy history, such as the therapy form (97.5 %) or time on PD (88.3 %). The assignment of such data may be relevant for patient stratification and understanding of clinical phenotypes.

**Table 2 Classification rates (%) for dialysis-related parameters**

| **Spectral range** | **Proteins 1800-1500 cm⁻¹** | | | | **Polysaccharides and phosphorous-containing molecules 1200-800 cm⁻¹** | | | |
|---|---|---|---|---|---|---|---|---|
| **Parameter** | **Model** | | | | | | | |
| **UrinVolOut (<1k/ ≥1k)** | **PCA-LDA** | | **PCA-MDA** | | **PCA-LDA** | | **PCA-MDA** | |
| **Treatment B, 4h** | | | | | | | | |
| Predicted | Actual | | Actual | | Actual | | Actual | |
| | <1k | ≥1k | <1k | ≥1k | <1k | ≥1k | <1k | ≥1k |
| <1k | 12 | 1 | 12 | 0 | 9 | 2 | 12 | 1 |
| ≥1k | 0 | 7 | 0 | 8 | 3 | 6 | 0 | 7 |
| Accuracy % | 100.00 | 87.50 | 100.00 | 100.00 | 75.00 | 75.00 | 100.00 | 87.50 |
| **Overall accuracy %** | | **93.8** | | **100.0** | | **75.0** | | **93.8** |

| **Treatment A+B, 4h** | | | | | | | | |
|---|---|---|---|---|---|---|---|---|
| Predicted | Actual | | Actual | | Actual | | Actual | |
| | <1k | ≥1k | <1k | ≥1k | <1k | ≥1k | <1k | ≥1k |
| <1k | 19 | 3 | 23 | 7 | 17 | 4 | 24 | 10 |
| ≥1k | 5 | 13 | 1 | 9 | 7 | 12 | 0 | 6 |
| Accuracy % | 79.17 | 81.25 | 95.83 | 56.25 | 70.83 | 75.00 | 100.00 | 37.50 |
| **Overall accuracy %** | | **80.2** | | **76.0** | | **72.9** | | **68.8** |

| **Parameter** | **Model** | | | | | | | |
|---|---|---|---|---|---|---|---|---|
| **UF (<-551/ ≥-551)** | **PCA-LDA** | | **PCA-MDA** | | **PCA-LDA** | | **PCA-MDA** | |
| **Treatment B, 4h** | | | | | | | | |
| Predicted | Actual | | Actual | | Actual | | Actual | |
| | <-551 | ≥-551 | <-551 | ≥-551 | <-551 | ≥-551 | <-551 | ≥-551 |
| <-551 | 8 | 3 | 9 | 0 | 9 | 1 | 10 | 0 |
| ≥-551 | 2 | 7 | 1 | 10 | 1 | 9 | 0 | 10 |
| Accuracy % | 80.00 | 70.00 | 90.00 | 100.00 | 90.00 | 90.00 | 80.00 | 70.00 |
| **Overall accuracy %** | | **75.0** | | **95.0** | | **90.0** | | **100.0** |

| **Treatment A+B, 4h** | | | | | | | | |
|---|---|---|---|---|---|---|---|---|
| Predicted | Actual | | Actual | | Actual | | Actual | |
| | <-551 | ≥-551 | <-551 | ≥-551 | <-551 | ≥-551 | <-551 | ≥-551 |
| **Spectral range** | **Proteins 1800-1500 cm⁻¹** | | | | **Polysaccharides and phosphorous-containing molecules 1200-800 cm⁻¹** | | | |
| <-551 | 15 | 3 | 15 | 3 | 18 | 5 | 18 | 4 |
| ≥-551 | 7 | 15 | 7 | 15 | 4 | 13 | 4 | 14 |
| Accuracy % | 68.18 | 83.33 | 68.18 | 83.33 | 81.82 | 72.22 | 81.82 | 77.78 |
| **Overall accuracy %** | | **75.8** | | **79.8** | | **77.0** | | **79.8** |

| **Parameter** | **Model** | | | | | | | |
|---|---|---|---|---|---|---|---|---|
| **ResidualCl (<3/ ≥3)** | **PCA-LDA** | | **PCA-MDA** | | **PCA-LDA** | | **PCA-MDA** | |
| **Treatment B, 4h** | | | | | | | | |
| Predicted | Actual | | Actual | | Actual | | Actual | |
| | <3 | ≥3 | <3 | ≥3 | <3 | ≥3 | <3 | ≥3 |
| <3 | 12 | 1 | 13 | 2 | 11 | 2 | 13 | 6 |
| ≥3 | 1 | 6 | 0 | 5 | 2 | 5 | 0 | 1 |
| Accuracy % | 92.31 | 85.71 | 100.00 | 71.43 | 84.62 | 71.43 | 100.00 | 14.29 |
| **Overall accuracy %** | | **89.0** | | **85.7** | | **78.0** | | **57.1** |

| **Treatment A+B, 4h** | | | | | | | | |
|---|---|---|---|---|---|---|---|---|
| Predicted | Actual | | Actual | | Actual | | Actual | |
| | <3 | ≥3 | <3 | ≥3 | <3 | ≥3 | <3 | ≥3 |
| <3 | 21 | 3 | 24 | 6 | 15 | 4 | 25 | 10 |
| ≥3 | 4 | 12 | 1 | 9 | 10 | 11 | 0 | 5 |
| Accuracy % | 84.00 | 80.00 | 96.00 | 60.00 | 60.00 | 73.33 | 100.00 | 33.33 |
| **Overall accuracy %** | | **82.0** | | **78.0** | | **66.7** | | **66.7** |

| **Parameter** | **Model** | | | | | | | |
|---|---|---|---|---|---|---|---|---|
| **DP Crea (<0.8/ ≥ 0.8)** | **PCA-LDA** | | **PCA-MDA** | | **PCA-LDA** | | **PCA-MDA** | |
| **Treatment B, 4h** | | | | | | | | |
| Predicted | Actual | | Actual | | Actual | | Actual | |
| | <0.8 | ≥0.8 | <0.8 | ≥0.8 | <0.8 | ≥0.8 | <0.8 | ≥0.8 |
| <0.8 | 9 | 1 | 10 | 0 | 11 | 1 | 11 | 0 |
| ≥0.8 | 2 | 8 | 1 | 9 | 0 | 8 | 0 | 9 |
| Accuracy % | 81.82 | 88.89 | 90.91 | 100.00 | 100.00 | 88.89 | 100.00 | 100.0 0 |
| **Overall accuracy %** | | **85.4** | | **95.5** | | **94.4** | | **100.0** |

| **Treatment A+B, 4h** | | | | | | | | |
|---|---|---|---|---|---|---|---|---|
| Predicted | Actual | | Actual | | Actual | | Actual | |
| | <0.8 | ≥0.8 | <0.8 | ≥0.8 | <0.8 | ≥0.8 | <0.8 | ≥0.8 |
| <0.8 | 21 | 3 | 24 | 1 | 20 | 4 | 23 | 3 |
| ≥0.8 | 4 | 12 | 1 | 14 | 5 | 11 | 2 | 12 |
| Accuracy % | 84.00 | 80.00 | 96.00 | 93.33 | 80.00 | 73.33 | 92.00 | 80.00 |
| **Overall accuracy %** | | **82.0** | | **94.7** | | **76.7** | | **86.0** |

Assignment for physiological parameters related to the PD-therapy (Table 2) may be of diagnostic value. The highest classification rates were achieved for assignment of dialysate-to-plasma creatinine ratio (100.0 %), and ultrafiltration (100.0 %) in the polysaccharide region, whereas the protein region separated best for residual urine output (100.0 %), and residual clearance (89.0 %) sample parameters.

Parameters such as ultrafiltration and dialysate-to-plasma creatinine ratio are currently determined by time extensive peritoneal equilibration test. Residual urine output and residual clearance require further collection of urine. It is desirable to obtain a similar information faster, e.g. with the spectroscopic method analyzing a peritoneal dialysis sample. Moreover, these parameters may give a good view on the technical performance of the dialysis.

**Table 3 Classification rates (%) for physiological parameters, e.g. biomarker concentrations**

| **Spectral range** | **Proteins 1800-1500 cm⁻¹** | | | | **Polysaccharides and phosphorous-containing molecules 1200-800 cm⁻¹** | | | |
|---|---|---|---|---|---|---|---|---|
| **Parameter** | **Model** | | | | | | | |
| **IL-8** | **PCA-LDA** | | **PCA-MDA** | | **PCA-LDA** | | **PCA-MDA** | |
| **Treatment B, 4h** | | | | | | | | |
| Predicted | | Actual | Actual | | | Actual | Actual | |
| | ≥3 | <3 | ≥3 | <3 | ≥3 | <3 | ≥3 | <3 |
| ≥3 | 6 | 2 | 11 | 3 | 6 | 2 | 11 | 3 |
| <3 | 5 | 7 | 0 | 6 | 5 | 7 | 0 | 6 |
| Accuracy % | 54.5 | 77.8 | 100.0 | 66.7 | 54.5 | 77.8 | 100.0 | 66.7 |
| **Overall accuracy %** | | **66.2** | | **83.3** | | **66.2** | | **83.3** |

| **Treatment A+B, 4h** | | | | | | | | |
|---|---|---|---|---|---|---|---|---|
| Predicted | Actual | | Actual | | Actual | | Actual | |
| | ≥3 | <3 | ≥3 | <3 | ≥3 | <3 | ≥3 | <3 |
| ≥3 | 14 | 6 | 25 | 7 | 16 | 2 | 26 | 8 |
| <3 | 13 | 7 | 2 | 6 | 11 | 11 | 1 | 5 |
| Accuracy % | 51.9 | 53.8 | 92.6 | 46.2 | 59.3 | 84.6 | 96.3 | 38.5 |
| **Overall accuracy %** | | **52.8** | | **69.4** | | **71.9** | | **67.4** |

| **Parameter** | **Model** | | | | | | | |
|---|---|---|---|---|---|---|---|---|
| **IL-6** | **PCA-LDA** | | **PCA-MDA** | | **PCA-LDA** | | **PCA-MDA** | |
| **Treatment B, 4h** | | | | | | | | |
| Predicted | Actual | | Actual | | Actual | | Actual | |
| | <200 | ≥200 | <200 | ≥200 | <200 | ≥200 | <200 | ≥200 |
| <200 | 13 | 2 | 13 | 7 | 11 | 2 | 13 | 7 |
| ≥200 | 0 | 5 | 0 | 0 | 2 | 5 | 0 | 0 |

| **Spectral range** | **Proteins 1800-1500 cm⁻¹** | | | | **Polysaccharides and phosphorous-containing molecules 1200-800 cm⁻¹** | | | |
|---|---|---|---|---|---|---|---|---|
| **Accuracy %** | **100.00** | **71.43** | **100.00** | **0.00** | **84.62** | **71.43** | **100.00** | **0.00** |
| **Overall accuracy %** | | **85.7** | | **50.0** | | **78.0** | | **50.0** |

| **Treatment A+B, 4h** | | | | | | | | |
|---|---|---|---|---|---|---|---|---|
| Predicted | Actual | | Actual | | Actual | | Actual | |
| | <200 | ≥200 | <200 | ≥200 | <200 | ≥200 | <200 | ≥200 |
| <200 | 25 | 3 | 27 | 4 | 26 | 2 | 25 | 2 |
| ≥200 | 3 | 9 | 1 | 8 | 2 | 10 | 3 | 10 |
| Accuracy % | 89.29 | 75.00 | 96.43 | 66.67 | 92.86 | 83.33 | 89.29 | 83.33 |
| **Overall accuracy %** | | **82.1** | | **81.5** | | **88.1** | | **86.3** |
| **Parameter** | **Model** | | | | | | | |
| **HSP3** | **PCA-LDA** | | **PCA-MDA** | | **PCA-LDA** | | **PCA-MDA** | |

| **Treatment B, 4h** | | | | | | | | |
|---|---|---|---|---|---|---|---|---|
| Predicted | Actual | | Actual | | Actual | | Actual | |
| | <0.5 | ≥0.5 | <0.5 | ≥0.5 | <0.5 | ≥0.5 | <0.5 | ≥0.5 |
| <0.5 | 9 | 0 | 9 | 0 | 7 | 2 | 9 | 0 |
| ≥0.5 | 0 | 11 | 0 | 11 | 2 | 9 | 0 | 11 |
| Accuracy % | 100.00 | 100.00 | 100.00 | 100.00 | 77.78 | 81.82 | 100.00 | 100.00 |
| **Overall accuracy %** | | **100.0** | | **100.0** | | **79.8** | | **100.0** |

| **Treatment A+B, 4h** | | | | | | | | |
|---|---|---|---|---|---|---|---|---|
| Predicted | Actual | | Actual | | Actual | | Actual | |
| | <0.5 | ≥0.5 | <0.5 | ≥0.5 | <0.5 | ≥0.5 | <0.5 | ≥0.5 |
| <0.5 | 11 | 5 | 13 | 5 | 12 | 4 | 13 | 3 |
| ≥0.5 | 6 | 18 | 4 | 18 | 5 | 19 | 4 | 20 |
| Accuracy % | 64.71 | 78.26 | 76.47 | 78.26 | 70.59 | 82.61 | 76.47 | 86.96 |
| **Overall accuracy %** | | **71.5** | | **77.4** | | **76.6** | | **81.7** |

| **Parameter** | **Model** | | | | | | | |
|---|---|---|---|---|---|---|---|---|
| **History of peritonitis** | **PCA-LDA** | | **PCA-MDA** | | **PCA-LDA** | | **PCA-MDA** | |
| **Treatment A+B, 4h** | | | | | | | | |
| Predicted | Actual | | Actual | | Actual | | Actual | |
| | Yes | No | Yes | No | Yes | No | Yes | No |
| Yes | 6 | 6 | 7 | 1 | 7 | 6 | 7 | 1 |
| No | 4 | 24 | 3 | 29 | 3 | 24 | 3 | 29 |
| Accuracy % | 60.00 | 80.00 | 70.00 | 96.67 | 70.00 | 80.00 | 70.00 | 96.67 |
| **Overall accuracy %** | | **70.0** | | **83.3** | | **75.0** | | **83.3** |

Furthermore, see Table 3, a clear correlation between PD spectral features and biomarkers indicating inflammation (IL-8, IL-6) and stress responses (HSP) could be shown. HSP could be well predicted using the protein as well as polysaccharide region (100.0 %). Prediction of IL-6 achieved an 88.1 % classification rate using the polysaccharide region. Discrimination between samples derived from patients with or without previous peritonitis achieved 83.3 % for the protein and polysaccharide spectral region. However, the overall accuracy could be further increased to 92.5 % by narrowing down the used spectral window to 850-800 cm⁻¹ within the polysaccharide spectral region using PCA-MDA (data not shown).

The concentration of biomarkers can be obtained with other methods and some individual biomarkers have been shown to correlate with peritoneal membrane status (e.g. IL-6 effluent concentrations and peritoneal small solute transport rate (PSTR)) (Lambie 2013). The spectrum based assignment, however, may provide an easier way of obtaining biomarker information.

Moreover, these data give a clear indication that the sample spectrum includes valuable information on the subject's physiological state (e.g. inflammation).

### Example 2 - Reference samples with outcome parameters

Based on the proof of concept shown in Example 1, it is reasonable that based on the sample spectrum a direct relation to the patient's disease state can be made without identification and quantification of individual biomarkers, provided a set of pre-assigned reference samples is given.

PD samples are available for a cohort of patients (approx. n=340), treated in Austria, mainly at the Medical University of Vienna between 2010 and 2019.

Out of the group approx. 85 experienced peritonitis (approx. 180 infection episodes in total), approx. 500 peritoneal equilibration tests were carried out (characterizing membrane state and/or membrane failure and transport characteristics) and approx. 400 technique failure events were registered.

In total, approx. 7800 samples are available. In some cases, multiple samples are available per patient (longitudinal profiles), in other cases only one sample per patient is available.

Based on these data, the reference sampled can be assigned to clinical outcome parameters. Thus, the data form the basis for training an algorithm computing a similarity value for a subject's PDE sample and said PDE sample may be assigned to a clinical parameter indicating
- the subject's risk of having or developing a peritonitis,
- the subject's risk of having or developing a peritoneal membrane deterioration and/or
- the risk of technical failure.

### Non-patent references

Aufricht, C., R. Beelen, M. Eberl, M. Fischbach, D. Fraser, A. Jorres, K. Kratochwill, M. LopezCabrera, P. Rutherford, C. P. Schmitt, N. Topley and J. Witowski (2017). "Biomarker research to improve clinical outcomes of peritoneal dialysis: consensus of the European Training and Research in Peritoneal Dialysis (EuTRiPD) network." Kidney Int 92(4): 824-835.
Gajjar, K., J. Trevisan, G. Owens, P. J. Keating, N. J. Wood, H. F. Stringfellow, P. L. Martin-Hirsch and F. L. Martin (2013). "Fourier-transform infrared spectroscopy coupled with a classification machine for the analysis of blood plasma or serum: a novel diagnostic approach for ovarian cancer." Analyst 138(14): 3917-3926.
Grunert, T., A. Monahan, C. Lassnig, C. Vogl, M. Muller and M. Ehling-Schulz (2014). "Deciphering host genotype-specific impacts on the metabolic fingerprint of Listeria monocytogenes by FTIR spectroscopy." PLoS One 9(12): e1 15959.
Grunert, T., R. Stephan, M. Ehling-Schulz and S. Johler (2016). "Fourier Transform Infrared Spectroscopy enables rapid differentiation of fresh and frozen/thawed chicken." Food Control 60: 361-364.
Grunert, T., M. Wenning, M. S. Barbagelata, M. Fricker, D. O. Sordelli, F. R. Buzzola and M. Ehling-Schulz (2013). "Rapid and reliable identification of Staphylococcus aureus capsular serotypes by means of artificial neural network-assisted Fourier transform infrared spectroscopy." J Clin Microbiol 51(7): 2261-2266.
Herzog, R., M. Boehm, M. Unterwurzacher, A. Wagner, K. Parapatics, P. Majek, A. C. Mueller, A. Lichtenauer, K. L. Bennett, S. L. Alper, A. Vychytil, C. Aufricht and K. Kratochwill (2018). "Effects of Alanyl-Glutamine Treatment on the Peritoneal Dialysis Effluent Proteome Reveal Pathomechanism-Associated Molecular Signatures." Mol Cell Proteomics 17(3): 516-532.
Herzog, R., L. Kuster, J. Becker, T. Gluexam, D. Pils, A. Spittler, M. K. Bhasin, S. L. Alper, A. Vychytil, C. Aufricht and K. Kratochwill (2017). "Functional and Transcriptomic Characterization of Peritoneal Immune-Modulation by Addition of Alanyl-Glutamine to Dialysis Fluid." Sci Rep 7(1): 6229.
Kratochwill, K., M. Boehm, R. Herzog, K. Gruber, A. M. Lichtenauer, L. Kuster, D. Csaicsich, A. Gleiss, S. L. Alper, C. Aufricht and A. Vychytil (2016). "Addition of Alanyl-Glutamine to Dialysis Fluid Restores Peritoneal Cellular Stress Responses - A First-In-Man Trial." PLoS One 11(10): e0165045.
Lambie, M., J. Chess, K. L. Donovan, Y. L. Kim, J. Y. Do, H. B. Lee, H. Noh, P. F. Williams, A. J. Williams, S. Davison, M. Dorval, A. Summers, J. D. Williams, J. Bankart, S. J. Davies, N. Topley and I. Global Fluid Study (2013). "Independent effects of systemic and peritoneal inflammation on peritoneal dialysis survival." J Am Soc Nephrol 24(12): 2071-2080.
Wiesenhofer, F. M., R. Herzog, M. Boehm, A. Wagner, M. Unterwurzacher, D. C. Kasper, S. L. Alper, A. Vychytil, C. Aufricht and K. Kratochwill (2019). "Targeted Metabolomic Profiling of Peritoneal Dialysis Effluents Shows Anti-oxidative Capacity of Alanyl-Glutamine." Frontiers in Physiology 9(1961).

## Claims

1. A method for analyzing a peritoneal dialysis analysis sample comprising the steps of
a) providing an analysis sample from a subject, wherein the subject is subjected to peritoneal dialysis and the analysis sample is based on the peritoneal dialysis effluent of said subject,
b) measuring a sample spectrum of analysis sample in a spectral range of from 4000 cm⁻¹ to 400 cm⁻¹ in a spectroscopy step applying Fourier-Transform-Infrared (FTIR) spectroscopy,
c) determining, in a comparison step, particularly in a computing step, a similarity value by comparing said sample spectrum to at least one reference spectrum obtained from at least one reference sample by measuring as defined in step b),
d) assigning the analysis sample to a clinical parameter based on said similarity value.

2. The method according to claim 1, wherein in step a) the analysis sample is the peritoneal dialysis effluent or wherein the analysis sample is prepared from the peritoneal dialysis effluent, in particular prepared by diluting the peritoneal dialysis effluent and/or freezing and thawing the peritoneal dialysis effluent.

3. The method according to any one of claims 1 to 2, wherein said spectrum in step b) is obtained by either applying FTIR transmission sample technique, wherein in step a) the sample is dried, or applying FTIR attenuated total reflection (ATR), wherein in step a) the sample is provided as liquid or dried.

4. The method according to any one of claims 1 to 3, wherein in step b) the sample spectrum is measured in at least one of the spectral ranges selected from the group consisting of from 3000 cm⁻¹ to 2800 cm⁻¹ (fatty acid region), from 1500 cm⁻¹ to 1200 cm⁻¹ (mixed region protein + fatty acid), from 1800 cm⁻¹ to 1500 cm⁻¹ (protein region) and from 1200 cm⁻¹ to 800 cm⁻¹ (polysaccharide region),
preferably in the range of from 1800 cm⁻¹ to 1500 cm⁻¹ or from 1200 cm⁻¹ to 800 cm⁻¹.

5. The method according to any one of the preceding claims, wherein in step c) the comparison step includes pre-processing of the sample spectrum.

6. The method according to any one of the preceding claims, wherein the similarity value is a numerical variable and/or categorical variable.

7. The method according to claim 6, wherein in the comparison step c) an algorithm is used to determine the similarity value in the comparison step, particularly in the computing step, wherein the algorithm includes application of a machine learning model.

8. The method according to claim 7, wherein the machine learning model is selected from the group consisting of neural networks, support vector machines, discriminant analysis, k-nearest neighbors algorithm, regression analysis, evolutionary-based algorithms, regression and decision tree learning, adaptive boosting, and combination thereof.

9. The method according to any one of claims 6 to 8, wherein the algorithm is based on principal component analysis-linear discriminate analysis (PCA-LDA) or principal component analysis-Mahalanobis discriminate analysis (PCA-MDA).

10. The method according to any one of claims 7 to 9, wherein the algorithm is trained with a set of reference spectra obtained by measuring a plurality of reference samples as defined in step b).

11. The method according to claim any one of the preceding claims, wherein the clinical parameter is selected from the group consisting of demographic parameters, physiological and pathological parameters including biomarker concentration, dialysis-related parameters, and outcome parameters.

12. The method according to claim 10, wherein the clinical parameter is an outcome parameter indicating
- the subject's risk of having or developing a peritonitis,
- the subject's risk of having or developing a peritoneal membrane deterioration and/or
- the risk of technical failure.

13. The method according to claim 10, wherein the clinical parameter is a dialysis-related parameter selected from the group of residual urine output, ultrafiltration, residual clearance, dialysate-to-plasma creatinine ratio, residual glomerular filtration rate, peritoneal small solute transport rate, mass transfer area coefficient, effective lymphatic absorption rate, transcapillary ultrafiltration rate, free water transport, and sodium dip.

14. The method according to claim 10, wherein the clinical parameter is a biomarker concentration, wherein the biomarker is selected from the group of proteins, metabolites, or biogenic amines.

15. The method according to claim 14, wherein the biomarker is a protein selected from the group of cytokines and chemokines, preferably interleukin-8 or interleukin-6, and heat shock proteins, preferably HSP72.
